# EUROPEAN PATENT SPECIFICATION

(11) **EP 0 601 071 B1**
(45) Date of publication and mention of the grant of the patent: **20.10.1999**
(21) Application number: 92919070.0
(22) Date of filing: 24.08.1992
(51) Int. Cl.: A61M 15/00

(54) **POWDER DISPENSER**
PULVERSPENDER
DISTRIBUTEUR DE POUDRE

(30) Priority: 26.08.1991 US 749912; 06.01.1992 US 817331
(43) Date of publication of application: 15.06.1994
(60) Divisional application: 97118730.7
(73) Proprietor: MINNESOTA MINING AND MANUFACTURING COMPANY, St. Paul, Minnesota 55133-3427 (US)
(72) Inventor: WHALEY, Ralph, D., Saint Paul, MN 55133-3427 (US); SHINNICK, Mark, S., Saint Paul, MN 55133-3427 (US); THIEL, Charles, G., Saint Paul, MN 55133-3427 (US); KRIEGL, DonGene, Saint Paul, MN 55133-3427 (US); REEDER, Thomas, W., Saint Paul, MN 55133-3427 (US); PATTOCK, Brian, M., Saint Paul, MN 55133-3427 (US); MATTILA, Robert, J., Saint Paul, MN 55133-3427 (US); TURGEON, Thomas, A., Saint Paul, MN 55133-3427 (US)
(74) Representative: VOSSIUS & PARTNER
(86) International application number: US9207156
(87) International publication number: WO9303785

(56) References cited:
- EP-A- 0 254 391
- EP-A- 0 424 790
- WO-A-90/07351
- WO-A-92/00771
- WO-A-92/04068
- GB-A- 2 144 997
- US-A- 4 524 769

## Description

The present invention relates generally to devices which facilitate the inhalation of powdered medicament.

Dose uniformity or accuracy is a problem associated with known dispensers which dispense powdered medicament. Generally, powdered medicaments tend to "pack" into unduly large agglomerates, particularly when the size of the particles is in the order of a few microns. If the medicament is delivered to a user in unduly large agglomerates, the large agglomerates tend to impact the tissue at the back of the user's throat and thus the medicament is prevented from reaching the user's lungs. Such impact also sometimes causes an uncomfortable "gag" reflex or coughing. Further, it is desirable to completely disperse the entire dose delivered to a user to maximise the respirable fraction and efficacy of the medicament delivered to the user and to avoid the attendant problems associated with large agglomerates of powder.

US-A- 4 046 146; US-A- 4 811 731 US-A- 2 587 215 and US-A- 4 524 769 all describe inhalators that are powered by the user's inhalation airflow. One problem associated with user powered dispensers is that the user's capacity to generate an effective airflow may be adversely affected by an ailment such as bronchial asthma. It is believed that the powdered medicament dispensers which rely upon user generated airflow tend to be inefficient, particularly when a user's capacity to generate an inhalation airflow is adversely affected by an ailment. Another problem associated with user powered dispensers is that different persons possess widely varying abilities to generate inhalation airflows. Thus, requiring a user to generate a considerable airflow may cause discomfort for some users.

US-A- 4 524 769 describes a device similar to a TURBOHALER T.M. dispenser which is generally available from Aktiebolaget Draco, of Lund, Sweden and is believed to be on sale in Europe. That device includes a perforated member with a plurality of perforations for each dosage of medicament. The TURBOHALER T.M. dispenser is a user powered inhalation device and is believed to suffer from the problems associated with user powered medicament dispensers mentioned above.

The art also has devices which facilitate the inhalation of powdered medicaments by the use of propellants to dispense and disperse the medicament. Dispensers which utilize a propellant are disclosed in US-A- 4 534 345 and US-A- 4 667 668. Other prior art dispensers use chlorofluorocarbon to deliver a pharmaceutically active compound. In these types of dispensers, the medicament is typically suspended within the chlorofluorocarbon propellant. While such dispensers are somewhat successful at delivering medicament to a user, it is not always desirable to use chlorofluorocarbon propellants for reasons including cost. Also, dispensers such as those mentioned above are believed to be complex to construct, inefficient or difficult to use.

GB-A-2 144 997 relates to a new dosage inhalator. The device comprises a first valve, second valve, dosing unit, perforated membrane and propellant container. The propellant container contains liquid or gasses propellants. The pressure within a particular propellant container only dissipates during use. A medicament is brought from a storage chamber into the perforations in the membrane using scrapers. The perforated membrane is then moved so that a part of the membrane area with its perforation loaded with medicament is introduced into the propellant passage. To actuate the device, a tilt lever is displaced by pushing on a trigger which opens the first valve and pressurizes a connecting passage and the dosing chamber at the expense of the pressure in the propellant container. In this state, the connecting passage is pressurized and the propellant passage is not pressurized. Next, the tilt lever is again displaced by pushing on a trigger which opens the second valve. The pressure within the connecting passageway and the dosing chamber must travel through the propellant passage before it communicates with the filled perforated membrane. The pressurization takes place after the filled perforated membrane has been moved to the opening of the propellant passage.

WO 90/07351 describes an oral inhaler having a product reservoir and a metering chamber which are connected through a plurality of small openings which allow the product to flow from the reservoir through the openings into the metering chamber.

WO 92/00771 which is a document according to Article 54(3) EPC relates to an inhaler having an inhalation passage with an air intake and a metering member.

WO 92/04068 which is a document according to Article 54(3) EPC describes an inhalator having a reservoir and a dozing device manually operated by means of a push-button with a dose chamber that receives a predetermined dose of the powdery substance.

It is the object of the present invention to provide an improved dry powder medicament dispenser.

This object is achieved with a dispenser according to the claims.

The present invention provides a simplified dry powder medicament dispenser which: (1) effectively and efficiently dispenses and disperses dry powder medicament into the inhalation airstream of a user even when that inhalation airflow is at a rate that is less than the rate of an average person, (2) affords a highly efficient airflow within the dispenser to dispense and disperse the dry powder medicament, (3) wastes very little medicament, and (4) provides repeatable dosage accuracy.

According to one aspect of the present invention, there is provided a dry powder medicament dispenser for delivering a dose of micronized particles of a dry powder medicament to the respiratory system of a user. The dispenser comprises a housing, and means for providing a packed, predetermined, agglomerated dose of the dry powder medicament in a dosage chamber which is dimensioned to hold the predetermined dose of the dry powder medicament. The dispenser also comprises an internal pressure generator for generating a pressure for forcibly expelling the predetermined dose from the dosage chamber and external of the housing in micronized, deagglomerated form suitable for inhalation therapy. The dispenser has an effectiveness in deagglomerating the packed dose which is generally independent of user inhalation rate.

The device preferably comprises a dosage member having surfaces defining the dosage chamber. The dosage chamber defines a loading axis which is generally perpendicular to the surfaces defining an end of the dosage chamber. The means for providing a packed, predetermined agglomerated dose preferably comprises a medicament reservoir for storing a supply of dry powder medicament, and an agglomerator within the medicament reservoir for providing a positive, orientation independent packing force for loading the dry powder medicament from the medicament reservoir into the dosage chamber under pressure sufficient to pack the dry powder into a reproducible dose.

Most preferably the agglomerator is mounted for movement across the dosage chamber so that the positive, orientation independent packing force has a component that is generally parallel to the loading axis. The component of the packing force that is generally parallel to the loading axis progressively increases as the agglomerator moves across at least a portion of the dosage chamber.

Preferably, the pressure generator comprises a pressure chamber having a pressure outlet and a medicament delivery passageway. The pressure chamber suddenly provides a substantial fluid pressure differential across the packed dose so that the fluid flows to deagglomerate the packed dose into a plurality of respirable particles. More preferably the pressure generator provides a fluid flow at the dose which is initially generally free of velocity but which reaches a maximum velocity soon after the fluid begins to flow. At the maximum velocity, the fluid flow is turbulent and deagglomerates the packed dose into a plurality of respirable particles.

Also preferably, the pressure generator provides a fluid pressure differential to progressively increasing portions of the dose. Most preferably, the pressure differential is provided across the dose by releasing a pressurized fluid generally immediately adjacent the dose.

In a preferred embodiment, the dosage member, medicament reservoir, delivery passageway and pressure outlet are mounted for relative movement between (1) a load orientation with the dosage chamber opening into the medicament reservoir, and (2) a delivery orientation with the dosage chamber situated between the pressure outlet and the delivery passageway. Between the load and delivery orientations, progressively increasing portions of the dosage chamber, medicament delivery passageway and pressure outlet become aligned.

The present invention may also be described as a method of dispensing multiple individual doses of a dry powder medicament comprising the steps of (1) providing micronized particles of the medicament within a housing, (2) packing the micronized particles into a predetermined, agglomerated dose in a dosage chamber, and (3) generating a fluid pressure to forcibly expel the predetermined dose from the dosage chamber and external of the housing in micronized, deagglomerated form suitable for inhalation therapy.

According to another aspect of the present invention there is provided a cartridge which (1) is adapted to be received in and cooperate with a dry powder medicament dispenser comprising pressurization and actuation mechanisms adapted to minimize or reduce the inputs or operations required of a user; (2) may be replaced in the dry powder medicament dispenser with another cartridge after it is depleted; (3) affords re-use of the dispenser with a number of different cartridges; (4) may be constructed to restrict tampering with the dry powder medicament; and (5) optionally includes a counter assembly which affords an estimate of the number of dosages of medicament available in the cartridge.

The present invention will be further described with reference to the accompanying drawing wherein like reference numerals refer to like parts in the several views, and wherein:
Figure 1 is a perspective fragmentary view of one embodiment of dispenser according to the present invention shown from its front side;
Figure 2 is an exploded perspective view of the dispenser of Figure 1 rotated approximately ninety-degrees and showing a piston for pressurizing the fluid pressure chamber;
Figures 3 through 5 are enlarged sectional views of the device of the present invention taken approximately along lines 3-3 of Figure 1 with portions broken away to show details and which sequentially illustrate the delivery of medicament to a user;
Figures 5A through 5C are enlarged fragmentary perspective views of parts of the dispenser of Figure 1 which sequentially illustrate movement of a dosage chamber into alignment with a pressure outlet passageway;
Figure 5D is an enlarged fragmentary schematic illustration of initial alignment of a dosage chamber with the outlet passageway that occurs during this sequence illustrated in Figures 5A-5C;
Figure 6 is a perspective view of portions of a dosing member and a pressurization chamber which are preferably included in the dispenser of Figure 1;
Figure 7 is a perspective view of loading blades on a support shaft which are preferably included in the dispenser of Figure 1;
Figure 7A is a computer simulation illustrating sequential movement of a loading blade relative to a dosage member;
Figure 8 is a sectional view of a second embodiment of a dispenser according to the present invention;
Figure 9 is a sectional view of a third embodiment of a dispenser according to the present invention;
Figure 10 is an exploded perspective view of a cartridge according to another aspect of the present invention;
Figure 11 is an enlarged perspective view of a partially assembled cartridge according to the present invention;
Figure 12 is an enlarged perspective view of some of the elements of the cartridge shown in Figure 10 taken at a different angle than that of Figure 10 to show various details;
Figure 13 is a perspective view of the elements shown in Figure 12 taken at a different angle than that of Figure 12 to illustrate various details;
Figure 14 is an enlarged perspective view of elements of the cartridge shown in Figure 10 taken at a different angle to show details of portions of a counter assembly;
Figure 15 is an enlarged sectional perspective view of the cartridge shown in Figure 10 with the elements assembled;
Figure 16 is an enlarged sectional perspective view of the cartridge shown in Figure 10 similar to Figure 15 with parts omitted to show detail;
Figure 17 is another enlarged sectional perspective view of the cartridge shown in Figure 10 with the elements assembled and with a cover omitted to show detail;
Figure 18 is an enlarged sectional view of a cartridge assembled from the elements shown in Figure 10 with elements broken away and omitted to illustrate detail;
Figure 19 is an enlarged sectional view of a cartridge assembled from the elements shown in Figure 10 and including a sealing element between a metering sleeve and a pressure chamber; and
Figures 20 and 21 are schematic representations of an example of a medicament dispenser for use with the cartridge of the present invention which sequentially illustrate the operation of the cartridge in conjunction with the dispenser.

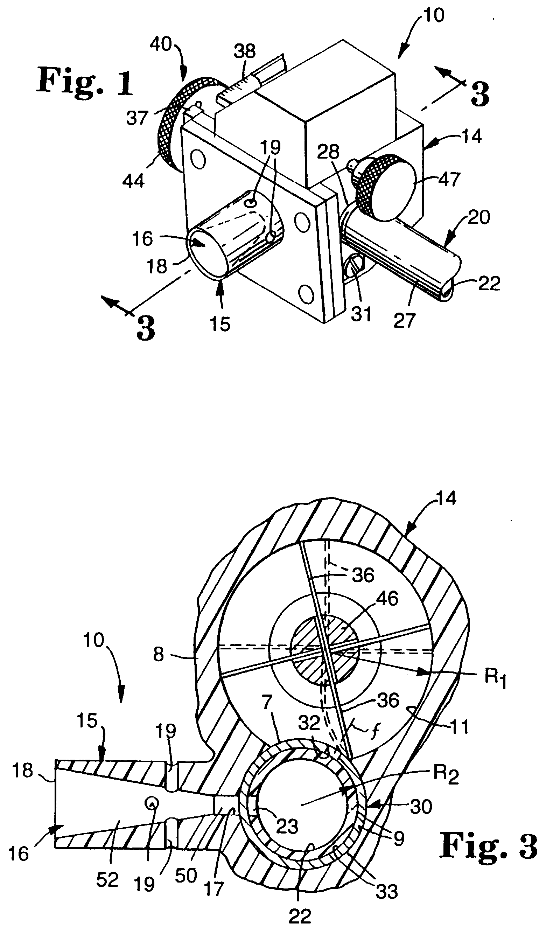

Referring now to Figures 1 through 7 of the drawing, there is shown one embodiment of dispenser according to the present invention, generally designated by the reference number 10. The dry powder medicament dispenser 10 delivers a dose of micronized particles of a dry powder medicament 12 to the respiratory system of a user.

The dispenser 10 has an effectiveness in deagglomerating the packed dose which is generally independent of patient inhalation rate. Thus, a respirable mass of the dry powder medicament may consistently be provided to a patient, even at reduced patient inhalation airflow rates.

The dry powder medicament dispenser 10 comprises a housing 14 including a mouthpiece portion 15. The dispenser 10 comprises means for providing a packed, predetermined, agglomerated dose of the dry powder medicament in a dosage chamber 32 which is dimensioned to hold the predetermined dose of the dry powder medicament.

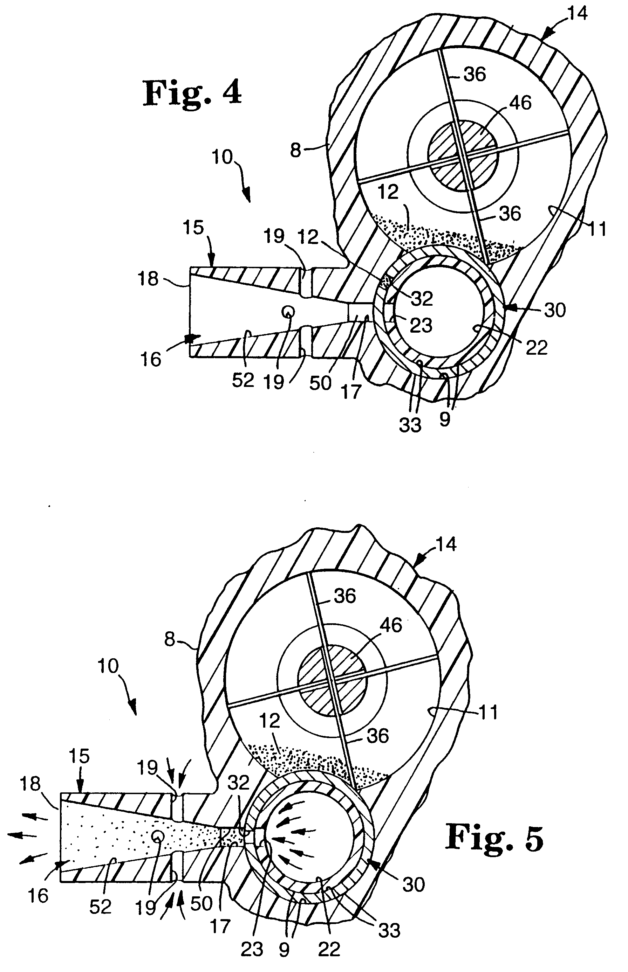

The device 10 comprises a dosage chamber 30 having surfaces defining the dosage chamber 32. The dosage chamber 30 defines a loading axis f (Figure 3) which is generally perpendicular to the surfaces defining an end of the dosage chamber 32.

The means for providing a packed, predetermined agglomerated dose preferably comprises a medicament reservoir 11 for storing a supply of dry powder medicament 12, and an agglomerator within the medicament reservoir 11 for providing a positive, orientation independent packing force for loading the dry powder medicament 12 from the medicament reservoir 11 into the dosage chamber 32 under pressure sufficient to pack the dry powder 12 into a reproducible dose.

Most preferably the agglomerator is mounted for movement across the dosage chamber 32 so that the positive, orientation independent packing force has a component that is generally parallel to the loading axis f. The component of the packing force that is generally parallel to the loading axis f progressively increases as the agglomerator moves across at least a portion of the dosage chamber. The agglomerator is described in greater detail below.

The dispenser 10 also comprises an internal pressure generator 21 for generating a pressure for forcibly expelling the predetermined dose from the dosage chamber 32 and external of the housing 14 in micronized, deagglomerated form suitable for inhalation therapy. Preferably, the pressure generator 21 provides a gas pressure differential to progressively increasing portions of the dose. This feature is described in detail below. The pressure differential is preferably provided by releasing a pressurized gas generally immediately adjacent the dose. As used herein, the phrase, "immediately adjacent the dose" means that the dispenser 10 is substantially free of an obstruction or hindrance (e.g. an unpressurized [relative to the pressure provided by generator 21] region of ambient air or a tortuous or labyrinth like path) which may result in a pressure drop between the position of the release of the pressurized gas and the dose or which may otherwise interfere with the communication of the pressure from the generator to the dose.

Also preferably, the pressure generator 21 generally instantaneously or suddenly provides a substantial gas pressure differential across the packed dose so that the gas flows to deagglomerate the packed dose into a plurality of respirable particles. As used herein, when it is said that the pressure generator suddenly provides a gas pressure differential across the packed dose, it is meant that the dispener is free of any obstruction or hindrance that would substantially delay or slow the communication of the pressure from the pressure generator 21 to the dose.

The powdered medicaments 12 may be from any suitable therapeutic category such as, but not limited to antibiotics, proteins/peptides, steroids, bronchodilators, anticholinergics, lipoxygenase inhibitors, PAF antagonists, potassium channel activators, mast cell stabilizers, bradykinin analogs, enkephalins or interleukin. For example not intended to be limiting, the powder say be leuprolide, albuterol, insulin, pirbuterol, beclomethasone, terbutaline, salmeterol, fluticasone, tiamcinolone, salbutamol, isoproterenol, epinephrine, fenoterol, formoterol, procaterol, pentamidine, calcitonin, ipratropium, oxitropium, budesonide or their pharmaceutically acceptable salts.

The dry powder medicament dispenser 10 is particularly suited for delivery of a predetermined dosage unit comprising a plurality of very fine particles having an average diameter as low as about 0.3 micrometers or even possibly less. Generally, by "very fine" or "micronized" particles, an average particle diameter is contemplated with a range of from about 0.3 micrometers to about 20 micrometers, and preferably from about 0.5 micrometers to about 6.0 micrometers. For example, a dose of the powdered medicament 12 say comprise 0.115 milligrams of a solid micronized albuterol sulfate powder having an average particle size of approximately 3 micrometers.

The housing 14 preferably has an outer surface 8 and inner surfaces. Those inner surfaces preferably comprise portions defining (1) a dosage member receiving chamber 9, (2) the medicament reservoir 11, (3) a medicament delivery passageway 16 extending between an injection inlet end 17 (e.g. a circular opening having a diameter of 1.57 millimeters (0.062 inches) formed by a bore in the housing 14) communicating with the dosage member receiving chamber and an outlet end 18 opening through the mouthpiece portion 15, (4) at least one air entrance passageway 19 having an inlet end opening through the outer surface 8 of the housing 14 and an outlet end opening into the medicament delivery passageway 16, (5) a gas pressure chamber 22, and (6) a pressure outlet passageway 23 with an inlet end communicating with the fluid pressure chamber 22 and an outlet end opening through the inner surface defining the dosage member receiving chamber generally opposite the injection inlet end 17 of the medicament delivery passageway 16. The housing 14 may be constructed from any suitable metal or polymeric saterial, such as but not limited to polyacetal, polyethylene, polypropylene, polycarbonates, or combinations thereof.

The medicament delivery passageway 16 preferably comprises a cylindrical turbulent flow portion 50 having a generally uniform cross section that is situated adjacent the injection inlet end 17 for affording a substantially turbulent flow of fluid from the fluid pressure chamber 22 to disperse the dry powder medicament 12 in the fluid. The medicament delivery passageway 16 optionally comprises a frusto-conical laminar flow portion 52 adjacent and diverging in cross sectional area toward the outlet end 18 of the medicament delivery passageway 16. The air entrance holes 19 open into the laminar flow portion 52 to afford laminar flow of fluid (preferably atmospheric air) and dry powder medicament in the laminar flow portion 52.

The inner surface portions defining the laminar flow portion 52 preferably diverge at an angle between approximately 15 and 30 degrees with respect to each other, preferably about 22 degrees. The embodiment of dispenser 10 shown in Figures 1 through 5 includes four circumferentially spaced cylindrical shaped air entrance holes 19 having axes generally transverse to or normal to the axis of the medicament delivery passageway 16.

The dispenser 10 includes the movable chamber means or dosage member 30 which may be a cylindrical tubular member having an inner diameter of 8.46 millimeters (0.333 inches) and a wall thickness of 0.46 millimeters (0.018 inches). Alternatively, the dosage member 30 may comprise a generally flat planar structure having a thickness of about 0.46 millimeters (0.018 inches).

In a preferred embodiment, the dosage member 30 comprises a dosage part having an outer sealing surface 33 and includes surfaces defining a dosage chamber 32 having the longitudinal axis f and extending through the dosage member 30 between spaced parts of the outer sealing surface 33 (see Figures 3-5). The dosage chamber 32 receives a dosage of dry powder medicament 12. The volume of the dosage chamber 32 is influenced by a variety of factors but is particularly influenced by the type of powdered medicament that is intended to be delivered. For example, when 0.115 mg. of albuterol sulfate is to be dispensed from the dosage chamber 32, the volume of the dosage chamber 32 should be approximately (1.45 X 10 (-5) cubic inches) 0.24 cubic millimeters . Also as an example, if the dosage chamber 32 is cylindrical, the dosage chamber may have a diameter of about 0.081 cm (0.032 inches) for a dosage member 30 that is 0.046 cm (0.018 inches) thick.

The cross-section of the dosage chamber 32 illustrated is circular to form a cylindrical passageway. Alternatively the dosage chamber 32 may have any suitable shape such as, but not limited to triangular, square, star, hexagonal, arcuate, polygonal, or any suitable shape form by combinations of straight and arcuate line segments. Preferably, there is a single dosage chamber 32 that provides a consistent dosage receiving volume which tends to receive consistent volumes of powdered medicament to thereby contribute to repeatable dosage accuracy.

The cross-section of the dosage chamber 32 may optionally be slightly smaller than the cross-section of the pressure outlet passageway 23 to afford complete expulsion of the medicament 12 from the dosage chamber 32, and to insure proper communication between the pressure outlet passageway 23 and the dosage chamber 32. For example, if the cross-section of the dosage chamber 32 is circular having a diameter of approximately (0.032 inches) 0.81 millimeters , then the cross-section of the pressure outlet passageway 23 may also be circular with a diameter of approximately (0.052 inches) 1.32 millimeters or (0.055 inches) 1.40 millimeters . Generally, the cross-section of the injection inlet 17 for the medicament delivery passageway 16 may have approximately the same or larger cross-sectional area than the cross-sectional area of the dosage chamber 32. For a circular cross-section of the dosage chamber 32 with a diameter of approximately 0.032 inches (0.81 millimeters), the cross-sectional area of the injection inlet 17 say also be circular with a diameter of approximately 0.062 inches (1.57 millimeters).

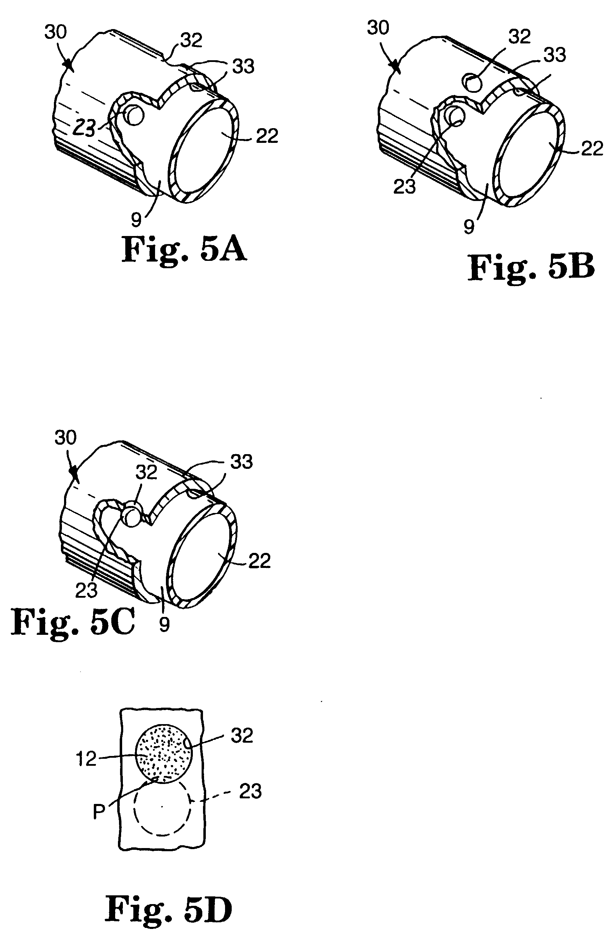

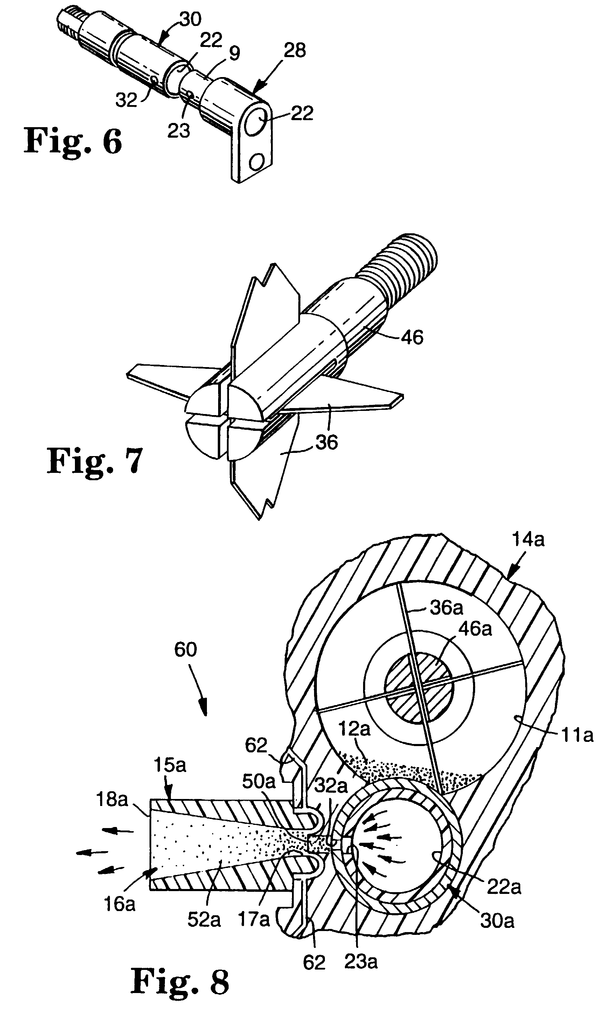

The dosage member 30 may be constructed from any suitable material such as, but not limited to, polymeric, plastic or metal materials or combinations thereof. The material used to construct the dosage member 30 and surfaces 9 should be sufficiently strong to resist deformation upon pressurization of a pressurization chamber 22. As best seen in Figures 5A-5C and 6, the dosage member 30 is preferably mounted adjacent portions of the inner surface 9 of the housing 14 which form the dosage member receiving chamber. Those portions of the inner surface 9 of the housing 14 which form the dosage member receiving chamber (see Figure 6) say comprise a part 28 which also forms portions of the fluid pressure chamber 22. Preferably the end of the part 28 adjacent gas pressure release aperture 23 has a closed end to form the pressurization chamber 22.

Means such as elastomeric sealing gaskets (not shown) may be provided to provide a seal between the dosage member 30 and the medicament reservoir 11 to prevent leakage or escape of powder 12 from the reservoir 11. Alternatively the means for preventing escape of powder 12 could comprise biasing means such as a screw for biasing the dosage member 30 into tight frictional engagement with the inner surface 9 of housing 14 adjacent outlet 7.

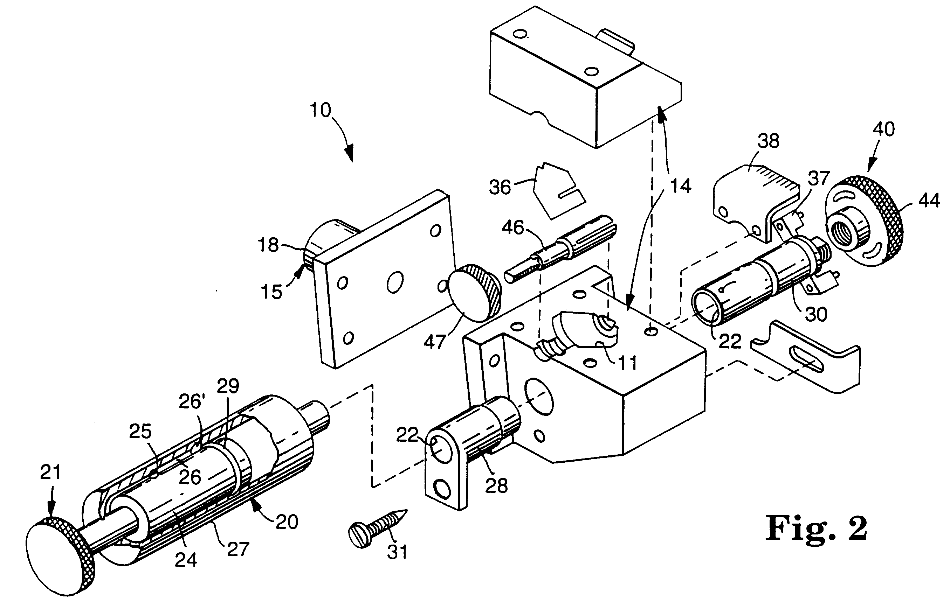

Preferably, the pressure generator 21 comprises a pressure chamber 22 having a pressure outlet 23 and the medicament delivery passageway 16. The pressure generator 21 (Figure 2) is provided for pressurizing gas (e.g. such as ambient air) within the gas pressure chamber 22 above ambient pressure and for retaining the gas pressure within the gas pressure chamber 22 above ambient pressure. The pressure outlet passageway 23 may be formed by a cylindrical bore in part 28. The part 28 may include a flange with an aperture to receive a screw 31 or any other suitable fastener to fix the part 28 in a proper position relative to the injection inlet opening 17 by firmly attaching the part 28 to the housing 14 so that the part 28 forms a portion of the inner surface 9 defining the dosage member receiving chamber. Alternatively the part 28 and the dispenser housing 14 may comprise a monolithic member such as an integrally molded member.

An example of a portion of the pressure generator 21 is illustrated in Figure 2 as a piston or plunger 24 having an O-ring 29 and a detent 25 adapted to be received in a channel or groove 26 in a gas pressure member housing 27. An open end of the part 28 is adapted to receive a Luer fitting (not shown) or any suitable adapter for forming an air-tight attachment between the gas pressurization member 20 and the part 28.

The groove 26 in the housing 27 has a first portion extending generally parallel to the axis of the fluid pressurization member housing 27 and a second locking portion 26 extending generally perpendicular to the first portion. To pressurize the gas pressure chamber 22, a user first sealingly covers the pressure outlet passageway 23 with surfaces 33 of the dosage member 30 (Figures 3 and 4) and then moves the piston 24 axially within the gas pressurization member housing 27 toward the housing 14 until the detent engages the second locking portion of the groove 26'. The piston 24 may then be rotated counterclockwise (relative to the housing 14) to move the detent 25 into the second locking portion of the groove 26. The pressure within the gas pressure chamber 22 biases the piston 24 away from the housing 27 and forces frictional engagement between the detent 25 and the locking portion of the groove 26' to retain the gas pressure within the gas pressure chamber 22 above ambient pressure.

While the means pressure generator 21 is shown in Figure 2 to include a piston 24, it should be noted that the pressure generator 21 say comprise any suitable means for pressurizing gas (e.g. atmospheric air) within the gas pressure chamber 22 above ambient pressure and for retaining the gas pressure within the gas pressure chamber 22 above ambient pressure including, but not limited to bellows, flexible bladders, or syringe/O-ring arrangements. Also, the means for locking the piston in a pressurization position may comprise any suitable means other than the described detent assembly.

The pressure generator 21 provides a gas flow at the dose from gas which is initially generally free of velocity but which reaches a maximum velocity soon after the gas begins to flow. At the maximum velocity, the gas flow is turbulent and deagglomerates the packed dose into a plurality of respirable particles. The pressure and volume within the pressurization chamber 22 provided by the generator 21 should be sufficient to completely expel all the powdered medicament 12 loaded into the dosage chamber 32.

While not intending to be bound by one theory, it is believed that the flow from a pressurized pressure chamber 22 to the medicament delivery passageway 16 may approximate flow through a nozzle or restriction such that there exists a critical pressure within the pressure chamber 22 which will provide a maximum velocity of flow within the medicament delivery passageway 16. Any additional pressure above the critical pressure will not result in gas velocities in the medicament delivery passageway 16 above the maximum velocity.

Sufficient pressure and initial volume within the gas pressure chamber 22 is believed to assure the maximum velocity of fluid escaping from the pressure chamber 22 into the medicament delivery passageway 16. Generally, the higher the velocity of the gas within the turbulence portion 50 the greater the turbulence in the turbulence portion 50. The greater the turbulence, the more the dispenser 10 disperses the powdered medicament 12 into smaller, more respirable particles. The turbulence results in high shear forces on the agglomerates of medicament 12 and causes collisions between the agglomerates of powdered medicament which tends to deagglomerate the large agglomerates into the desired primary particles. For example, the gas pressure chamber 22 may be pressurized to a pressure of about 7.65 Kg/cm² (108.8 pounds per square inch) absolute.

For a given size of dosage chamber 32, the pressure of gas within the gas pressure chamber 22 sufficient to expel the powdered medicament from the dosage chamber 32 is controlled by several factors, such as the size of the pressure outlet passageway 23, the initial volume of the gas chamber 22 before pressurization and the speed with which the dosage chamber 32 is moved across the pressure outlet passageway 23. Generally, for optimal dispensing, it is believed that a user should move the dosage chamber 32 across the pressure outlet passageway 23 as fast as possible. Optionally a biasing means such as a spring may be used to move the dosage chamber 32 across the pressure outlet 23.

Preferably, the entire cross sectional area of the dosage chamber 32 ultimately overlaps with the pressure outlet 23 and the total elapsed time between the time that (1) the dosage chamber 32 begins to open into the pressure outlet 23, until (2) the entire cross-sectional area of the dosage chamber 32 overlaps with the pressure outlet 23, is about four milliseconds.

When the pressure chamber 22 is pressurized, the medicament delivery passageway 16 remains at ambient or atmospheric pressure and there exists the potential to place a pressure differential across the packed dose when the dosage chamber 32 begins to overlap or open into the outlet 23. An example of the pressure generator 21 providing a substantial gas pressure differential across the packed dose is that the pressure within the pressure chamber 22 should be at least about 1.1 times the ambient air pressure (the pressure in medicament delivery passageway 16). Preferably the pressure within the pressure chamber 22 should be at least about twice the ambient air pressure. The volume of pressureized gas within chamber 22 should be enough to provide gas flow during at least the time between when (1) the dosage chamber 32 begins to open into the pressure outlet 23, and (2) the entire cross-sectional area of the dosage chamber 32 overlaps the pressure outlet 23.

A manually-activatable means 40 (Figures 1 and 2) or optionally an automatic means mounts the dosage member 30 with at least portions of the outer sealing surface 33 in sealing engagement with the inner surface 9 defining the dosage member receiving chamber for movement from (1) a load position with the dosage chamber 32 generally aligned with the outlet opening 7 of the medicament reservoir 11, to (2) a delivery position with the dosage chamber 32 extending between the outlet end of the pressure outlet passageway 23 and the injection inlet end 17 of the medicament delivery passageway 16.

Preferably, the dosage member is in direct communication with the reservoir. By direct communication, it is understood that the dosage chamber 32 is directly exposed to the reservoir without intervention of any filter or any plurality of small orifices. In this way, it is possible to more optimally compact the entire amount of the drug into the chamber 32.

The means 40 may comprise an activation knob 44 (Figures 1 and 2) operatively connected to the dosage member 30 and having a flange 37 immovably affixed thereto. The housing 14 may have a stop flange 38 immovably affixed thereto. Rotation of the knob 44 causes the flange 37 to move relative to the stop flange 38. The stop flange 38 has surfaces adapted to abut the knob flange 37 when the dosage member 30 is moved between the load and delivery positions. Abutment between the one side of the stop flange 38 and the flange 37 ensures that the dosage chamber 32 moves to the correct position for dispensing relative to the pressure outlet passageway 23 and injection inlet 17, and abutment between the other side of the stop flange 30 and the flange 37 ensures that the dosage chamber 32 is in a position to receive powdered medicament 12 from the reservoir 11.

At the load position (Figure 3) the inner surface 9 defining the dosage member receiving chamber seals the end of the dosage chamber 32 opposite the medicament reservoir opening 7, and the outer sealing surface 33 of the dosage member part 32 seals shut the outlet end of the pressure outlet passageway 23. Portions 33 of the dosage member 30 seal shut the outlet end of the pressure outlet passageway 23 during an initial part of the movement of the dosage member 30 from the load position (e.g. Figure 3) to the delivery position (e.g. Figure 5). During a final part of the movement from the load position to the delivery position, progressively increasing portions of the pressure outlet passageway 23, the dosage chamber 32, and the injection inlet 17 move into alignment to afford dispersion of the dry powder medicament 12.

When (1) the dosage member 30 is positioned at the load position so that medicament 12 from the medicament reservoir 11 may be moved into the dosage chamber 32, (2) the generator means 21 is activated to pressurize fluid within the pressure chamber 22, and (3) the dosage member 30 is then moved to the delivery position while a user is inhaling air through the air entrance passageway 19, pressurized fluid will pass from the pressure chamber 22 through the pressure outlet passageway 23 and discharge the powdered medicament 12 from the dosage chamber 32 into the air stream being inhaled by the user through the air entrance passageway 19.

While the dosage chamber 32 has been described as moving relative to the housing 14 and the reservoir 11, it should be noted that any of the dosage member 32, medicament reservoir 11, delivery passageway 16 or the pressure outlet 23 may be mounted for movement so long as (1) in the load position or orientation, the dosage chamber 32 opens into the medicament reservoir 11, and (2) in the delivery position or orientation, the dosage chamber 32 is situated between the pressure outlet 23 and the delivery passageway 16. Optionally, the delivery passageway 16 may be omitted.

Figures 5A, 5B and 5C sequentially illustrate portions of the pressure outlet passageway 23 and the dosage chamber 32 moving into alignment. Figure 5D illustrates the dosage chamber 32 as it initially opens into the pressure outlet passageway 23.

When the dosage chamber 32 initially opens into the pressure outlet 23, a pressure differential is provided across the dose. There is generally very little or no pressure loss between the position of release of the pressurized fluid within chamber 22 and the dose. The pressure differential provided across the dose by the pressure within the pressure chamber 22 and ambient pressure in the delivery passageway 16 is preferably the maximum pressure differential that may be provided across the dose for those pressures. Thus, pressure within the pressure chamber 22 is efficiently used by the dispenser 10. Alternatively, the pressure provided by the generator which forcibly expels the dose from the dosage chamber 32 say be provided by a vacuum across the dose.

The feature of the present invention wherein the pressure outlet passageway 23 is situated directly or immediately adjacent the dosage chamber 32 during dispensing of the medicament 12 is believed to contribute to complete dispersion of the medicament 12 within the dosage chamber 32. This "eclipse" motion of the dosage chamber 32 relative to the pressure outlet passageway 23 while pressure chamber 22 is pressurized is believed to effectively remove and deagglomerate the powder in dosage chamber 32. The eclipse motion of the dosage chamber relative to the pressure outlet is believed to afford complete, efficient dispersion of the dry powder medicament, and provides a cloud of respirable sized powdered medicament that is easily inhaled by a user, even at an inhalation airflow that is less than the rate at which an average person say inhale.

The portion P (See Figure 5D) of the dosage chamber 32 that initially opens into the pressure outlet passageway 23 and the injection inlet 17 is believed to be "blasted" from the dosage chamber 32 with the remaining powder following shortly thereafter. The pressure immediately at the location of the powder 12 is believed to effectively disperse and dispense the powder 12 from the dosage chamber 32 to the delivery passageway 16.

Dispensing a powdered medicament 12 in the manner according to the present invention reduces the amount of pressure required to completely dispense the powder 12. The pressurization chamber 22 need only be pressurized with enough pressure to (1) deagglomerate the powder 12, and (2) expel the medicament 12 from the dosage chamber 32 and into the medicament delivery passageway 16. The pressure within the pressurization chamber 22 is not required to transmit all of the powder into the mouth of the user. The user's inhalation through air entrance hole 19 subsequently draws the dispersed powder completely through the medicament delivery passageway 16 and into the lungs of the user.

### POWDER LOADING ASSEMBLY

The dry powder 12 tends to adhere to the walls of the medicament reservoir 11 and to form unduly large agglomerates. The dispenser 10 according to the present invention utilizes a novel powder loading assembly that contributes to consistent dosage accuracy. The powder loading assembly includes means for providing a packed, predetermined, agglomerated dose of the dry powder medicament in a dosage chamber 32 which is dimensioned to hold the predetermined dose of the dry powder medicament. The means cause transfer of the dry powder medicament 12 from the medicament reservoir 11 to the dosage chamber 32 in the dosage member 30 when the dosage member 30 is in the load position.

The means for providing a packed, predetermined agglomerated dose preferably comprises the medicament reservoir 11, and an agglomerator within the medicament reservoir 11 for providing a positive, attitude or orientation independent packing force for loading the dry powder medicament 12 from the medicament reservoir 11 into the dosage chamber 32 under pressure sufficient to pack the dry powder 12 into a reproducible dose.

When the dispenser 10 is used to dispense micronized particles of a dry powder drug 12, the micronized particles tend to resist flow from the medicament reservoir 11 into the dosage chamber 32. The packing force is described as positive because it is independent of or greater than ambient forces such as van der Waals forces between the micronized particles, or the effect of gravity on the micronized particles. The packing force is said to be orientation independent because the powder loading assembly will reproducibly load the medicament 12 into the dosage chamber 32 even in an upside down or inverted orientation.

The effect of the packing means of the present invention is to pack the powdered medicament into the dosage chamber. Packing the dosage chamber is believed to contribute to repeated, consistent dosage accuracy as the chamber 32 is consistently packed with the same amount of powder.

Preferably, the structure comprises flexible loading blades 36 each having proximal and distal 39 ends and a leading surface between the proximal and distal ends. The powder loading assembly includes at least one and preferably four flexible powder loading blades 36 mounted on a shaft or core 46 having generally the same axis as the axis of the medicament reservoir 11. Means 40 (Figure 2) drives the blade 36 across the surfaces of the medicament reservoir 11.

The blade turning shaft 46 is rotated by a powder loading knob 47. The blades 36 are constructed from any suitable flexible material including but not limited to polymers, metals or polyesters. Rotation of the knob 47 while the dosage chamber 32 opens into the medicament reservoir causes revolving movement of the blades 36 that scrapes medicament 12 from the walls of the reservoir 11, simultaneously loads the dosage chamber 32 with a dosage of dry powder medicament 12 and also tends to agitate the powder 12 to break the powder 12 into smaller agglomerates which are more readily loaded into the dosage chamber 32.

The shaft or core 46 moves the flexible blades 36 along a predetermined path within the medicament reservoir 11 with the leading surface leading and the distal end 39 of the blade 36 moving along a portion of the inner surface defining the medicament reservoir 11 during a first portion of the predetermined path (Figure 3, solid line). During a second portion (Figure 3 dashed lines) of the predetermined path, the distal end 39 of the blade 36 moves along a portion of the outer sealing surface 33 of the dosage member 30 which results in progressively increasing bending of the blade 36 to form the leading surface into a convex surface and to move the leading surface progressively closer to the outer sealing surface 33 of the dosage member 30.

When the distal end 39 of the blade 36 is described as moving along a portion of the outer sealing surface of the dosage member, it is herein contemplated that a thin layer of powder 12 e.g. 0.025 cm (0.01 inches) may be present between the distal end of the blade 36 and the dosage member 30.

The medicament reservoir 11 is generally cylindrically concave and has a medicament reservoir axis which defines a reservoir radius R1. The outer surfaces of the dosage member 30 along with its axis define a radius R2. The core 46 mounts the proximal ends of the blades 36 for revolving movement around the medicament reservoir axis. The portion of the outer sealing surface 33 of the dosage member 30 along which the distal end of the blade 36 moves is cylindrically concave about an axis generally parallel to the medicament reservoir axis R1.

The distance between the axes of the blade 36/medicament reservoir 11 and the dosage member 30 is less than the sum of the radii of the dosage member 30 and the medicament reservoir 11 (R1 + R2) to afford interference between the powder loading blade 36 and the dosage member 30 during the second portion of the predetermined path so that the powdered medicament 12 is loaded into dosage chamber 32 in a direction that is generally in the direction of the longitudinal or loading axis of the dosage chamber 32.

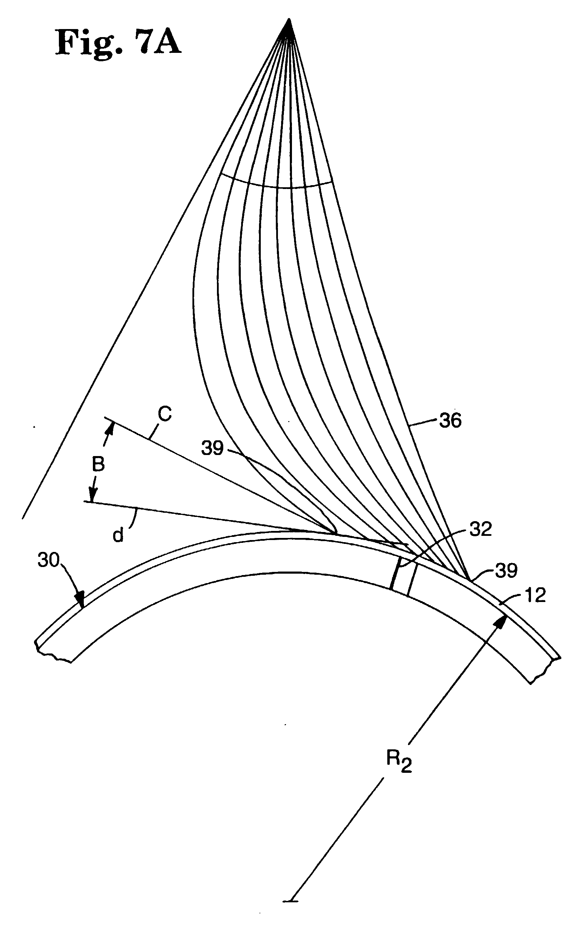

Figure 7A depicts a computer simulation of the powder loading assembly according to the present invention. To generate the computer simulation, a powder layer 12 of 0.025 cm (0.01 inches) was assumed to be present between the blade 36 and the dosage member 30. The simulation illustrates the position of the blade 36 for equal increments of rotation of core 46. When the flexible blade 36 engages the dosage member 30, the blade 36 will bend. At the line of contact between the blade 36 and the sealing surface of the dosage member 30 (or a thin layer of powder 12 directly adjacent thereto), there is an imaginary blade tangent line c that is tangent to the leading surface of the blade 36 and an imaginary dosage member tangent line d tangent to the outer sealing surface of the dosage member 30. The blade tangent line c and the dosage member tangent line d form a blade tangent angle Beta therebetween which progressively decreases as the blade 36 moves along the sealing surface and across the dosage chamber 32.

The loading assembly according to the present invention tends to beneficially pack the powdered medicament 12 into the dosage chamber 32. Just after the blade 36 encounters the dosage member 30, the action is similar to a rolling motion. As the blade moves across the dosage chamber 32, the blade tangent angle Beta progressively decreases so that the powdered medicament 12 is loaded into dosage chamber 32 in a direction that is generally along the loading axis f of the dosage chamber 32 (e.g. shown in Figure 3 by the dashed lines, and Figure 7). Forcing the powdered medicament in a direction generally along the axis f of the dosage chamber 32 is believed to repeatedly load the chamber with substantially uniform mounts of medicament 12 to thereby contribute to dosage accuracy. As an example which is not intended to be limiting, the dosage member 30 may have a radius R2 of approximately 0.475 cm (0.187 inches) the medicament reservoir 11 may have a radius R1 of approximately 0.81 cm (0.32 inches), and the distance between the axes of the medicament reservoir 11 and the dosage member 30 may be 1.19 cm (0.47 inches). The powder loading blades 36 may have a length from the axis of the medicament reservoir 11 of approximately 0.8 cm (0.315 inches) and are constructed from a flexible material such as polyester to ensure that the blade will deflect to the position shown in Figure 3 by the dashed lines.

Preferably, at least one of the loading blades 36 has a raking surface comprising a V-shaped notch (Figure 7) in its distal end 39 that disperses agglomerates of the dry powder medicament 12 into smaller particles and separates the dry powder 12 from the walls of the medicament reservoir 11.

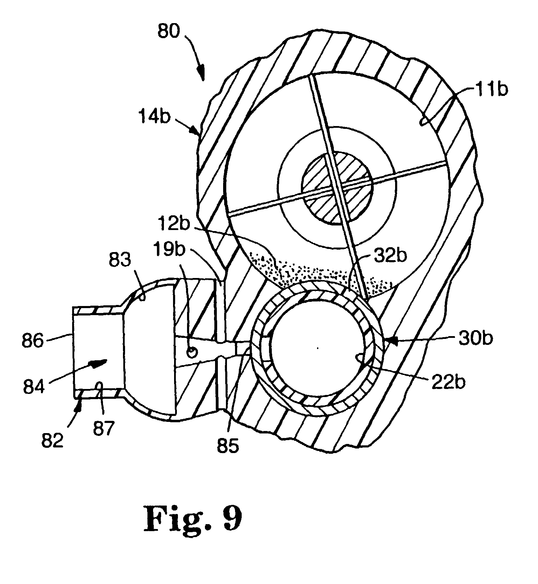

Figure 8 illustrates a second alternative embodiment of a dispenser according to the present invention designated by the reference character 60 which has many parts that are essentially the same as the parts of the dispenser 10 and which have been identified by the same reference number to which the suffix "A" has been added.

Like the dispenser 10 described in Figures 1 through 5, the dispenser 60 shown in Figure 8 comprises a housing 14A defining a medicament reservoir 11A which is adapted for storing micronized dry powder medicament 12A, and a mouthpiece 15A having surfaces defining a medicament delivery passageway 16A having an injection inlet opening 17A and an outlet opening 18A for passage of a respirable dose of the dry powder 12A for subsequent delivery to a user. A gas pressure chamber 22A adapted to be pressurized above ambient pressure and a movable dosage member 30A having surfaces defining a dosage chamber 32A are also provided.

Unlike the dispenser 10, the dispenser 60 includes arcuate air entrance holes 62 which are positioned to afford airflow into the medicament delivery passageway 16A at an angle relative to the axis of the passageway which is much less than ninety-degrees, preferably approximately zero (0) degrees. The position configuration of the air entrance holes 62 shown in Figure 8 is believed to provide a more laminar flow of air to a user.

Figure 9 illustrates a third alternative embodiment of dispenser according to the present invention designated by the reference character 80 which has many parts that are essentially the same as the parts of the dispenser 10 and which have been identified by the same reference number to which the suffix "b" has been added.

Like the dispenser 10 described in Figures 1 through 5, dispenser 80 shown in Figure 9 comprises a housing 14B defining a medicament reservoir 11B for storing micronized dry powder medicament 12B, air entrance holes 19B, a mouthpiece portion 82, a gas pressure chamber 22B adapted to be pressurized above ambient pressure and a movable dosage member 30B having surfaces defining a dosage chamber 32B are also provided.

Unlike the dispenser 10, the inner surfaces of the dispenser 80 include a medicament delivery passageway 84 having an injection inlet opening 85 and an outlet opening 86 for passage of a respirable dose of the dry powder 12B for subsequent delivery to a user. The medicament delivery passageway 84 has a deceleration portion 83 which has a larger cross sectional area than its turbulent and laminar flow portions and which is preferably semispherically shaped. The deceleration portion affords rapid expansion and loss of kinetic energy of the powder 12 after it exits the dosage chamber 32b. The deceleration portion 83 restricts passage of unduly large agglomerates of medicament 12 to deter such agglomerates from becoming entrained in the back of the user's throat. The semispherical chamber 83 may have a radius between about 0.51 cm 1.78 cm (0.2 and 0.7 inches)and may be connected to a cylindrical passageway 87 which may have a radius between about 0.51 cm and 1.27 cm (0.2 and 0.5 inches).

### OPERATION

Using the device of the present invention one can perform a method of dispensing multiple individual doses of a dry powder medicament 12 comprising the steps of (1) providing micronized particles of the medicament 12 within a housing 14, (2) packing the micronized particles 12 into a predetermined, agglomerated dose in a dosage chamber 32, and (3) generating a fluid pressure to forcibly expel the predetermined dose from the dosage chamber 32 and external of the housing 14 in micronized, deagglomerated form suitable for inhalation therapy.

More particularly, the operation will now be explained using the dispenser 10 as an example. First, a user should position the dosage chamber 32 in a position to receive medicament 12 from the reservoir 11. The user may rotate knob 44 until flange 37 abuts one side of stop flange 38. This position is designed to place the dosage chamber 32 in the position shown in Figure 3. At this position, the user may rotate knob 47 to rotate blades 36 to thereby load powdered medicament 12 into the dosage chamber 32.

Either before or after loading the dosage chamber 32 with medicament 12, the gas pressure chamber 22 should be pressurized by pushing the piston 24 toward the housing 14 and rotating the piston 24 to move the detent 25 into the groove 26' to retain the pressure in the chamber 22. During pressurization of chamber 22, the dosage member 30 may be in any position, such as either the position illustrated in Figure 3 or the position illustrated in Figure 4, as long as the dosage chamber 32 does not open into the pressure outlet passageway 23 and injection inlet 17.

Once the dosage chamber 32 is loaded with powdered medicament 12 and the pressurization chamber 22 is pressurized, the user may then actuate the dispenser 10 by rotating the knob 44 as quickly as possible until flange 37 abuts the other side of stop flange 38. This position is designed to place the dosage chamber 32 in the position shown in Figure 5. As the powder filled dosage chamber 32 initially begins to open into the pressure outlet passageway 23, the pressure within the gas pressure chamber 22 is highly concentrated over the portion of the dosage chamber 32 that is initially opening into the pressure outlet passageway 23 (Figure 5D). Such concentration of pressure at the location of the powder 12 is believed to effectively disperse and dispense the powder 12 from the dosage chamber 32 to the delivery passageway 16.

Either immediately before, during or just after moving the dosage chamber 32 to the position shown in Figure 5, the user should inhale creating an air flow through air entrance passageway 19 and through portions of medicament delivery passageway 16. The inhalation is preferably generally synchronous with rotation of knob 44 to ensure delivery of the powder 12 to the user as is appropriate with the pulmonary target. However, it is believed that some time may pass before inhalation without an unduly large loss of dispenser efficiency. The pressure within the gas pressure chamber 22 is generally not sufficient to transport the powder 12 completely into the mouth or nose of a user as is appropriate with the pulmonary target. However, once the powder 12 is expelled from dosage chamber 32, a user should ultimately inhale to effectively transmit the powder 12 to the user's lungs.

### CARTRIDGE ASSEMBLY

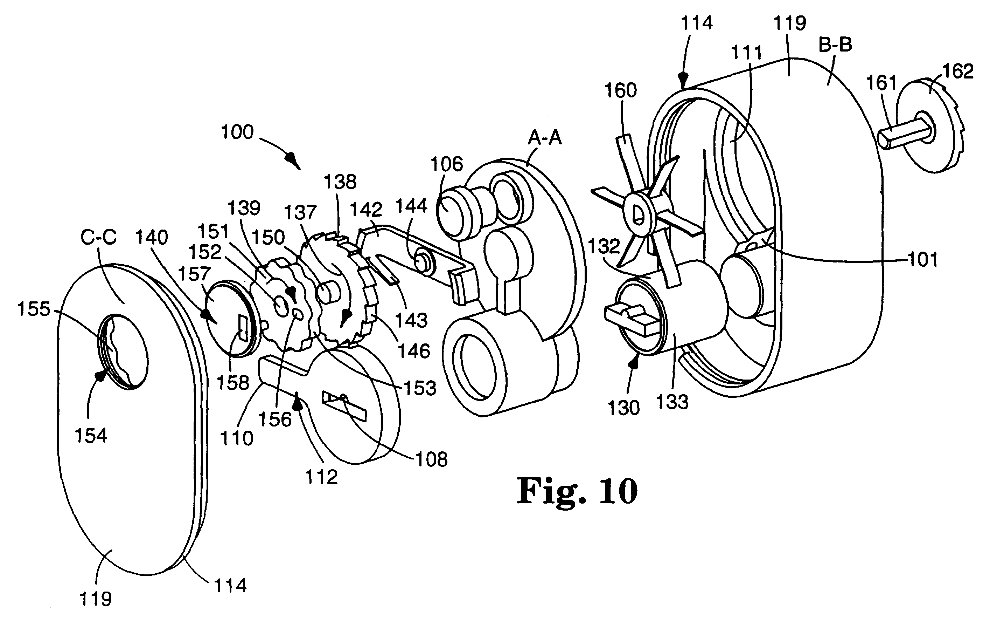

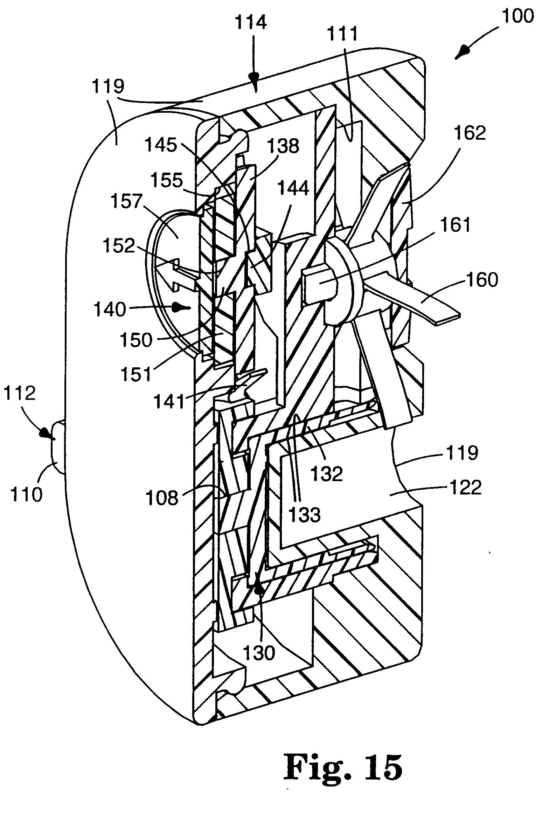

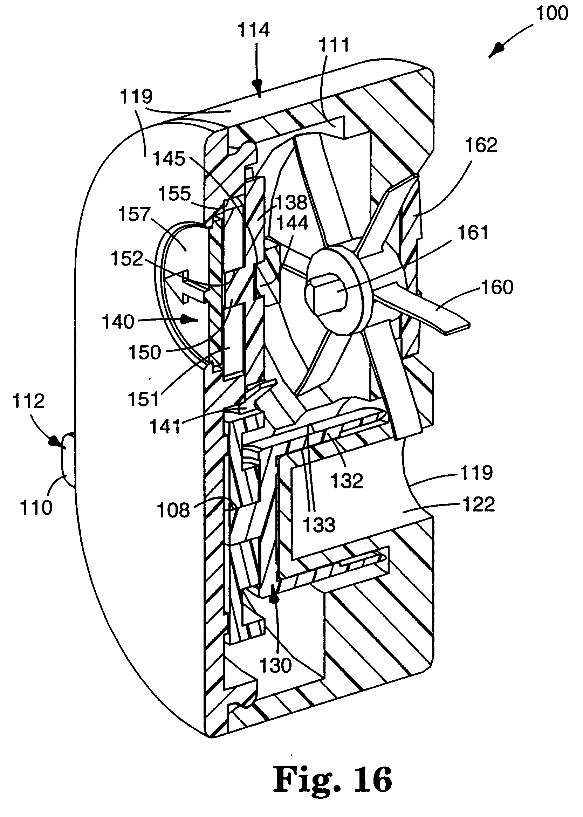

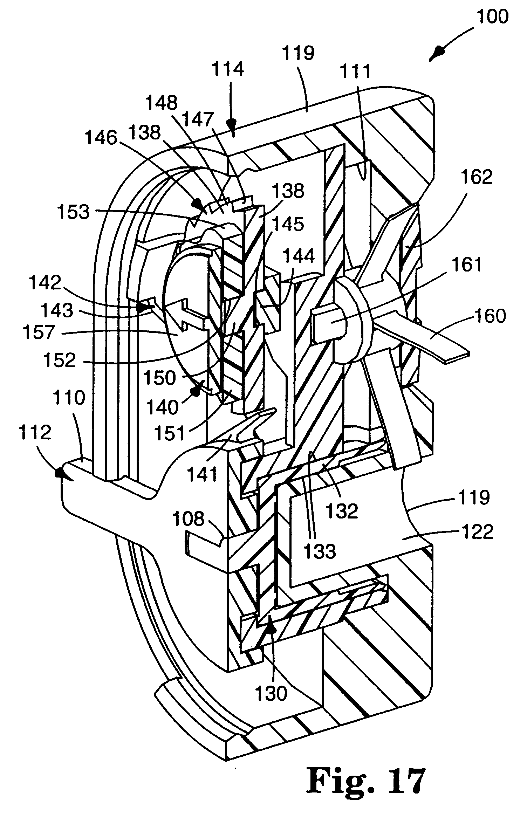

Referring now to Figures 10 through 21 of the drawing, there is shown an embodiment of cartridge which is generally designated by the reference character 100.

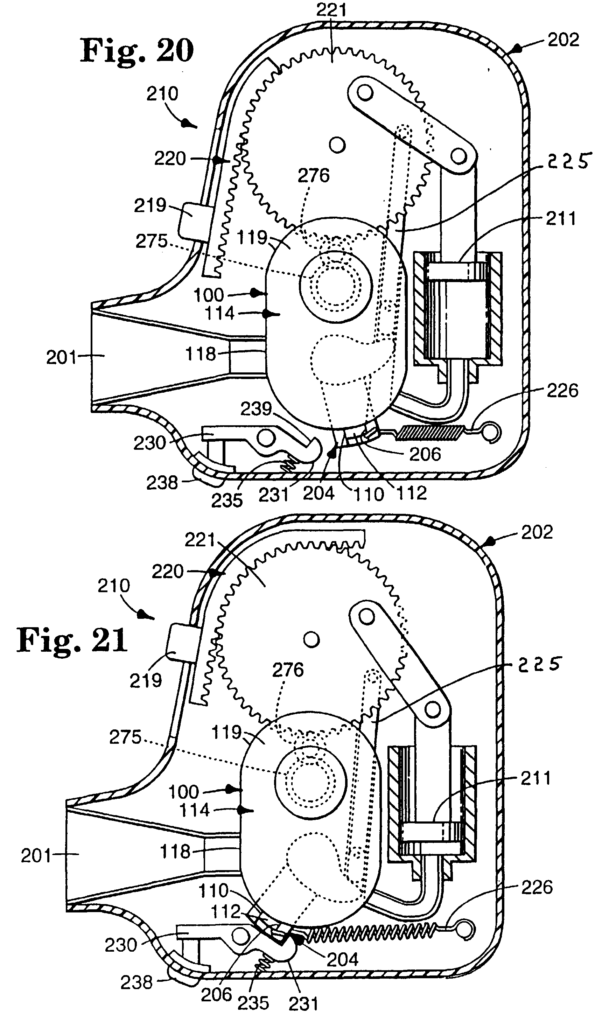

The cartridge 100 is received in a dry powder medicament dispenser 200 (Figures 20 and 21) having a mouthpiece portion 201, and actuation means 210 including pressurization means 211 to be explained in greater detail later.

The cartridge 100 may be constructed to be relatively inexpensive and disposable. Thus, when the medicament within a cartridge 100 is depleted, the cartridge 100 is replaced with a different cartridge and the depleted cartridge is disposed of. Such an arrangement affords re-use of the relatively expensive dispenser 200 with a number of different cartridges 100.

The cartridge 100 comprises a cartridge housing 114 including outer surfaces 119 adapted to be received in the dry powder dispenser 200. The housing 114 may be constructed from a material similar to the material used to construct the housing 14 and is preferably constructed from any suitable material approved by the U.S. Food and Drug Administration for medical purposes, such as the polycarbonate grade # HP-1 LEXAN T.M., generally available from General Electric of Pittsfield Massachusetts.

Figure 10 illustrates three separate major elements that are included in the assembly forming the cartridge 100. The major elements include a cartridge base BB, an intermediate portion AA and a cover CC. Preferably the parts AA, BB and CC may be press and/or snap fit together to form parts of the cartridge 100. Additionally, the cover CC say be adhesively adhered to the base BB to restrict tampering and access to medicament within cartridge 100. It should be noted that while the housing 114 is described as preferably comprising three major parts AA, BB and CC, alternatively the housing 114 may be comprised of fewer or additional major parts.

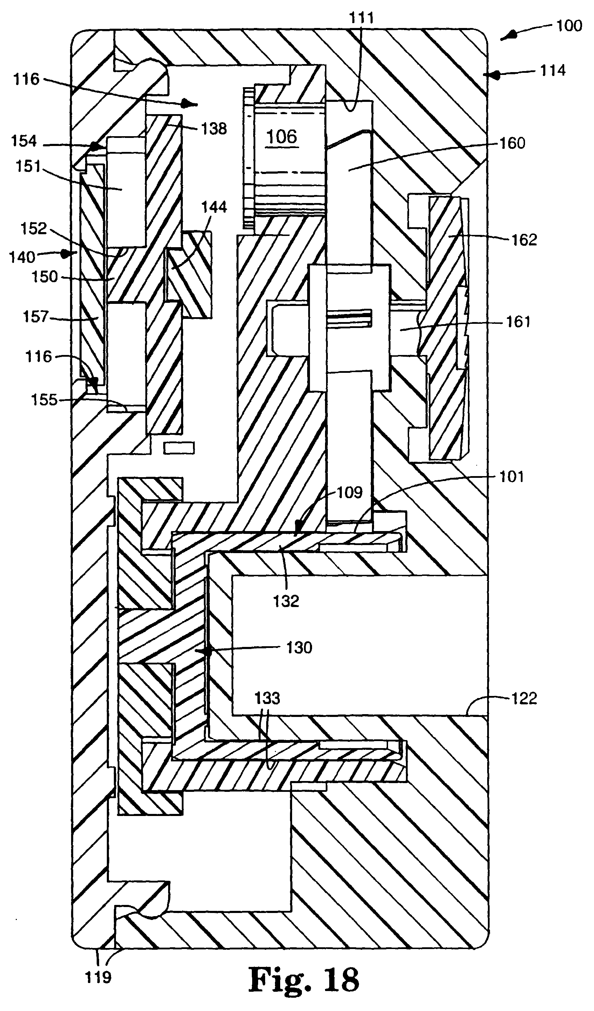

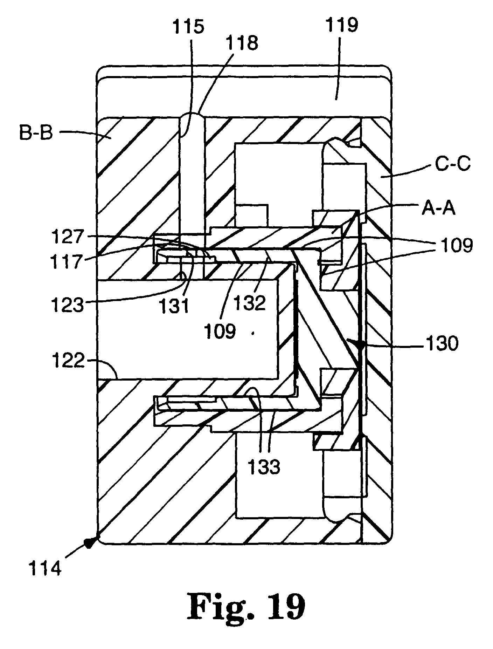

The housing 114 Includes inner surfaces comprising a dosage member receiving chamber 109 (Figure 19), a counter assembly receiving cavity 116 (Figure 18), and a medicament reservoir 111 for storing dry powder medicament (e.g. the medicament 12 described above in the description of the dispenser 10). The medicament reservoir 111 has a loading aperture 101 (Figure 18) communicating with the dosage member receiving chamber 109. As an example not intended to be limiting, the medicament reservoir 111 may be semi-cylindrical shaped as shown in Figures 10, and 15-18 and may include an outer diameter of about 19.3 millimeters and a width of about 2.03 millimeters. A desiccant plug 106 may be attached to intermediate portion AA to cover an access aperture to reservoir 111. The desiccant plug 106 removes moisture from the medicament 12. It should be noted that powder say also be initially loaded into the reservoir 111 through aperture 101.

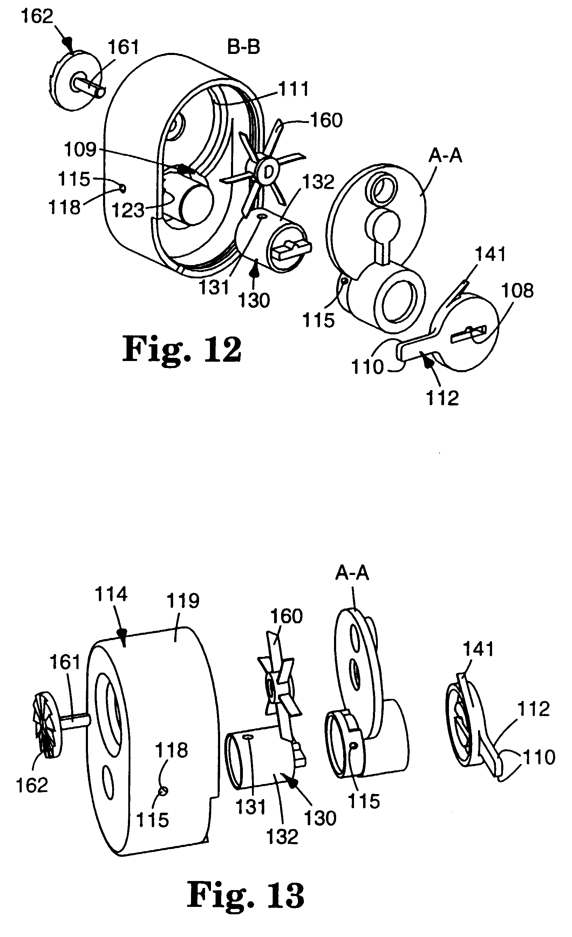

The inner surfaces of the housing 114 also include a medicament release bore 115 (e.g. a cylindrical bore having a diameter of about 1.6 millimeters and a length of about 3.2 millimeters) extending between an outlet end 118 at the cartridge housing 114 outer surfaces 119 and an injection inlet end 117 communicating with the dosage member receiving chamber 109. As shown in Figures 20 and 21, the outlet end 118 communicates with the mouthpiece portion 201 of the medicament dispenser 200. Also, as shown in Figures 12, 13 and 19, the medicament release bore 115 extends through portions of both major parts AA and BB.

Additionally, the inner surfaces of the housing 114 include a pressure chamber 122 (e.g. a cylindrical chamber having a diameter of the inner surfaces of the chamber of approximately 5.82 millimeters) that opens to the outer surfaces 119 of the housing 114. The pressure chamber 122 is operatively connected to the pressurization means 211 of the medicament dispenser 200 by, for example, a suitable sealing means (e.g. an elastomeric or rubber ring or washer, not shown) press fit between the housing 114 and the dispenser 200.

The pressure chamber 122 is pressurized by the pressurization means 211 of the medicament dispenser 200. For example, the pressurization means 211 may comprise a piston/cylinder arrangement as shown in Figures 20 and 21. Alternatively, the pressurization means 211 may comprise any suitable pressurization means for pressurizing fluid such as but not limited to bellows or flexible bladders.

Figure 19 illustrates that the inner surfaces of the housing 114 include a pressure release bore 123 (e.g. a cylindrical bore having a diameter of about 1.6 millimeters and having a length of about 1.01 millimeters) having a first end communicating with the pressure chamber 122 and a second end opening through the inner surface defining the dosage member receiving chamber 109 generally opposite the injection inlet end 117 of the medicament release bore 115.

The cartridge 100 includes a dosage member 130 mounted within the dosage member receiving chamber 109. The dosage member 130 includes a cylindrical, tubular dosage part 132 having sealing surfaces 133 and having a dosage chamber 131 extending through the dosage part 132 between spaced parts of the sealing surfaces 133. The dosage member 130 may be constructed of a material similar to the material used to construct the dosage member 30 and is preferably a material approved by the U.S. Food and Drug Administration for medical purposes such as, but not limited to, Grade #M90 SELCON T.M. generally available from HOECHST CELANESE.

The dosage chamber 131 receives a dosage of dry powder medicament from the medicament reservoir 111. The volume of the dosage chamber 131 is influenced by a variety of factors similar to the factors affecting the volume of the dosage chamber 32, particularly the type of medicament that is intended to be delivered. As an example not intended to be limiting, the dosage member 130 may comprise a sleeve with dimensions similar to those given in the example of the dosage member 30 described above.

The cross-section of the dosage chamber 131 may vary similar to the cross-section of the dosage chamber 32 mentioned above. Preferably, there is a single dosage chamber 131 to provide a consistent dosage volume which tends to receive consistent amounts of powdered medicaments to thereby contribute to dosage accuracy.

Like the dispenser 10, in the cartridge 100, means such as elastomeric or rubber sealing gaskets (not shown) may optionally be disposed to provide a seal between the dosage member 130 and the medicament reservoir 111, generally at the aperture 101.

Additionally, Figure 19 illustrates a sealing means added to the elements of the cartridge 100 shown in Figure 10. The sealing means comprises, for example, a biasing rubber or elastomeric member 127 attached between the dosage member 130 and the pressure release bore 123/pressure chamber 122 to provide a seal between the pressure release bore 123/pressure chamber 122 and the dosage member 130. The sealing means 127 may be simultaneously injection molded with the remaining portions of the pressure chamber 122 portion of the housing or may be adhered thereto with an appropriate adhesive. As shown in Figure 19, the sealing means 127 includes a bore extending therethrough so that the dosage chamber 131 and medicament release bore 115 communicate with the pressure release bore 123.

The cartridge 100 also includes an actuation arm 112 connected to the dosage part 132 and having portions extending beyond the outer surfaces 119 of the cartridge housing 114. The actuation arm 112 includes surfaces 110 adapted to be manipulated by the actuation means 210 of the medicament dispenser 200. The actuation arm 112 may be connected to the dosage member in any suitable manner such as by a snap fit with a detent groove 108 to afford proper orientation of the arm 112 relative to the dosage member 130.

The actuation arm 112 moves the dosage member 130 from (1) a load position (e.g. Figure 21) with the dosage chamber 131 opening into the loading aperture of the medicament reservoir 111, to (2) a delivery position (Figure 20). The movement between the load and delivery positions of the dosage member 130 relative to the reservoir 111, injection inlet 117 and the pressure release bore 123, is similar to the movement between the load and delivery positions of the dosage member 30 relative to the reservoir 11, gas pressure release aperture 23 and the injection inlet 17 of the dispenser 10 shown in Figures 3, 5, and 5A through 5D as discussed above. Like the dispenser 10, in the cartridge 100, at the load position, a portion of the inner surfaces defining the dosage member receiving chamber 109 seals the end of the dosage chamber 131 opposite the medicament reservoir 111, and a portion of the sealing surfaces 133 of the dosage member 130 seals shut the second end of the pressure release bore 123. At the delivery position, the dosage chamber 131 extends between the second end of the pressure release bore 123 and the injection inlet end 117 of the medicament release bore 115.

Portions of the inner surface defining the dosage member receiving chamber 109 seal shut both ends of the dosage chamber 131 and portions of the sealing surface 133 of the dosage member part 132 seal shut the second end of the pressure release bore 123 during an initial part of the movement of the dosage member 130 from the load position to the delivery position, and progressively increasing portions of the pressure release bore 123, the dosage chamber 131 and the injection inlet 117 move into alignment during a final part of the movement of the dosage member 130 from the load to the delivery position.

When (1) the dosage member 130 is positioned at the load position so that powdered medicament 12 from the medicament reservoir 111 flows into the dosage chamber 131, (2) the pressure chamber is pressurized, and (3) the dosage member 130 is then moved from the load position to the delivery position, pressurized gas will pass from the pressure chamber 122 through the pressure release bore 123 and discharge the powdered medicament 12 from the dosage chamber 131 into the medicament release bore 115.

The cartridge assembly 100 say optionally include a counter assembly 140 mounted within the counter assembly receiving cavity 116 for calculating the number of times the actuation arm 112 moves the dosage member 130 from the load position to the delivery position and back to the load position. Such a calculation affords an estimate of the number of dosages of medicament remaining in the medicament reservoir 111.

The counter assembly 140 comprises the actuation arm 112 having a counter assembly drive rod 141, the cartridge housing 114 having indicating means such as numerals (not shown) on its outer surfaces, a restraining assembly 142 comprising a leaf pawl 143 mounted within the counter assembly receiving cavity 116.

The counter assembly further includes the cartridge housing 114 having a ratchet wheel hub 144 (e.g. disposed on the restraining assembly 142), a ratchet wheel 138 having an axis, axially centered hub bearing surfaces 145, and a plurality of teeth 146 at its periphery. For example, the ratchet wheel 138 has a diameter of about 13.41 millimeters.

Each of the teeth 146 have a shoulder surface 147 and a release surface 148. The ratchet wheel bearing surfaces 145 are journaled on the ratchet wheel hub 144, and the ratchet wheel 138 is mounted within the counter assembly receiving cavity 116 for rotation relative to the cartridge housing 114. When the dosage member 130 moves from the delivery position to the load position, the drive rod 141 engages a shoulder surface 147 of a ratchet wheel tooth 146 to sequentially move the ratchet wheel 138 in a first rotational direction 137 (Figure 10) relative to the cartridge housing 114 to record the delivery of a dosage of medicament, and engagement between the leaf pawl 143 and a shoulder surface 147 of a ratchet wheel tooth 146 arrests movement of the rachet wheel 138 relative to the cartridge housing 114 in a direction opposite to the first rotational direction 137 when the drive rod 141 moves out of engagement with the shoulder surface 147 and along a release surface 148 of another ratchet wheel tooth 146 when the dosage member 130 moves from the load to the delivery position.

The counter assembly 140 may comprise only the rachet wheel 138 described above, when for example there are only a few dosages of medicament within the reservoir 111. In this example, the ratchet wheel 138 includes indicia such as an arrow (not shown) thereon for cooperating with the numerals (not shown) on the outer surface 119 of the housing to calculate the number of times the actuation arm 112 moves the dosage member 130 from the load position to the delivery position and back to the load position. However, preferably the counter assembly further comprises reduction means, particularly when the medicament reservoir 111-holds a large number of dosages (e.g. over 25).

The following described reduction means is believed to afford counting of up to about 210 dosages of medicament. The reduction means comprises the ratchet wheel 138 having an axially offset drive rib 150, an eccentric orbital gear 151 having an axis and bearing surfaces 152 adapted to receive the drive rib 150, and radially outwardly extending gear teeth 153. For example, the ratchet wheel 138 is constructed from any suitable material such as a polycarbonate or an acetal or an acetate, and has a pitch diameter of about 0.536 cm (0.211 inches).

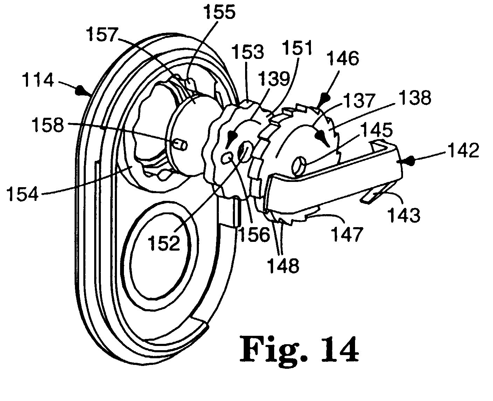

The reduction means also comprises the inner surfaces of the cartridge housing 114 having an annulus 154 (Figure 14) including an annulus axis, and radially inwardly extending gear teeth 155 for engaging the gear teeth 153 of the eccentric orbital gear 151. For example, the pitch diameter of the annulus 154 is approximately 0.58 cm (0.23 inches).

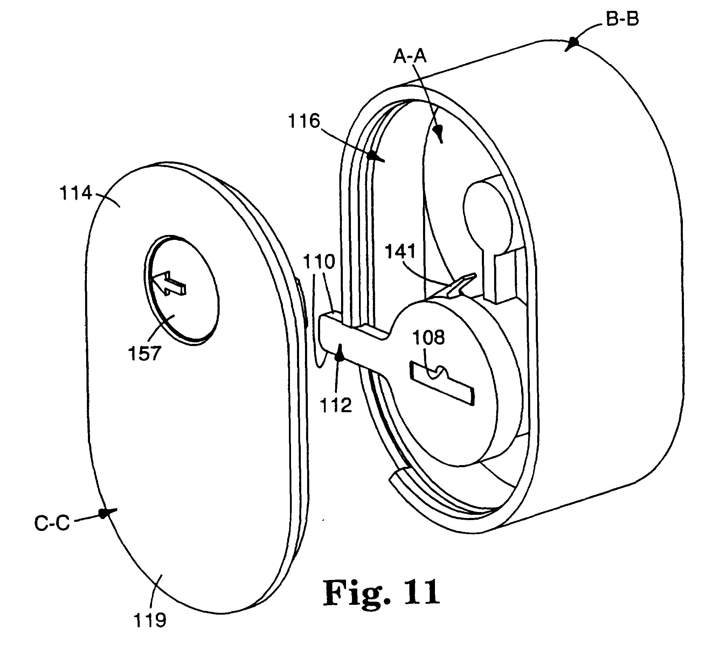

When the ratchet wheel 138 is driven in the first direction 137, the eccentric orbital gear teeth 153 engage the annulus gear teeth 155, and the eccentric orbital gear 151 moves in a second rotational direction 139 (Figure 10) generally opposite the first rotational direction 137. Optionally, the reduction means further includes the eccentric orbital gear 151 having in axially offset drive bearing slot surfaces 156, and a cover plate 157 having indicating indicia means (e.g. the arrow shown in Figure 11) thereon, and a drive finger 158 received in the bearing slot surfaces 156 of the eccentric orbital gear 151. In operation, when the eccentric orbital gear 151 moves relative to the annulus 154, the bearing slot surfaces 156 move the cover plate 157 in generally the second rotational direction 139 to thereby move the indicating indicia means (e.g. the arrow) on the cover plate 157 relative to the indicating means (e.g. the numerals not shown) on the outer surfaces 119 of the cartridge housing 114.

The bearing slot surfaces 156 are larger than the drive finger 158, and the drive finger say move within the slot surfaces 156. Thus, the cover plate 157 translates the eccentric motion of the eccentric orbital gear 151 into increments of generally circular motion so that the indicia means (e.g. the arrow) rotates in a defined, aesthetically pleasing circular path relative to the indicating means (e.g. the numerals) on the outer surfaces 119 of the cartridge housing 114.

The cartridge 100 also includes a powder loading assembly similar to the powder loading assembly for the dispenser 10 described above. The cartridge 100 and powder loading assembly afford storage of powdered medicament 12 in a reservoir 111 that (1) may be stirred or agitated after the cartridge 100 is delivered to the ultimate user, and (2) may be stored remote from the dispenser 200 prior to its use.

The powder loading assembly is a means for transferring dry powder medicament from the medicament reservoir 111 to the dosage chamber 131 in the dosage member 130 when the dosage member 130 is in the load position.

The powder loading assembly comprises at least one and preferably several flexible blades 160 having proximal and distal ends. The blades 160 may be constructed, for example, from a polyetherimid material such as grade #1000 ULTEM generally available from General Electric of Pittsfield, Massachusetts. Like the blade 32 of the dispenser 10, the blade 160 separates agglomerates of the dry powder medicament 12 into smaller agglomerates and separates the dry powder from walls of the medicament reservoir 111.

The powder loading assembly also includes a blade shaft 161 connected to the blade 160, and a one-way gear clutch 162 connected to the blade shaft 161 and adapted to be engaged by the actuation means 210 of the medicament dispenser 200. For example, the actuation means of the medicament dispenser 200 may include a complementary spring loaded gear clutch 275 which engages the one-way gear clutch 162 to drive the blade(s) 160 in a predetermined powder loading direction, and which releases from the one-way gear clutch 162 when the spring loaded gear clutch 275 is rotated in a direction opposite the powder loading direction.

Also like the blade 36 in the dispenser 10, when the blade 160 in cartridge 100 is mounted within the medicament reservoir and is driven by the actuation means 210 of the medicament dispenser 200, the blade 160 moves along first and second predetermined paths within the medicament reservoir 111. During a first part of the predetermined path, the leading surface of the blade 160 leads and the distal end of the blade 160 moves along a portion of the inner surface defining the medicament reservoir 111. During a second portion of the predetermined path, the distal end of the blade 160 moves along and contacts a portion of the outer sealing surface 133 of the dosage member 130 to progressively increasingly bend the blade 160 to form the leading surface into a convex surface and to move the leading surface progressively closer to the portion of the outer sealing surface 133 of the dosage member 130 adjacent the dosage chamber 131 to pack powdered medicament into the dosage chamber 131 (see Figure 7A).

### OPERATION OF THE CARTRIDGE

The operation of the cartridge will now be described with reference to the preferred embodiment 100 and with reference to an example of a dispenser 200. Figures 20 and 21 sequentially illustrate the operation of the cartridge 100 in conjunction with the dispenser 200.

The cartridge 100 shown in Figures 20 and 21 is generally identical to the cartridge 100 shown in Figures 10 through 19 except that the position of the actuation arm 112 in the load and delivery positions is generally lower in Figures 20 and 21 than the position of the actuation arm 112 in Figures 10 through 19. The actuation arm 112 is shown in this manner to better illustrate the operation of the actuation means 210 of the dispenser 200.

The cartridge 100 is received in the dispenser 200 which includes a housing 202 including a mouthpiece portion 201, pressurization means 211 and actuation means 210. The actuation means is preferably a mechanical assembly which minimizes or reduces the inputs or operations required from a user. However, it should be noted that the invention comprises the cartridge 100 and thus, the actuation means may comprise a variety of different actuation means from the strictly manual actuation similar to that described with respect to the dispenser 10 to completely automatic actuation means or combinations of manual and automatic actuation means.

The actuation means 210 includes, for example, the actuation means shown in Figures 20 and 21. Those actuation means include a cammed fork member 204 (shown in Figures 20 and 21 by dashed lines) having surfaces 206 adapted to receive the manipulation surfaces 110 of the actuation arm 112. The cammed fork member 204 is pivotally mounted on the housing 202 of the dispenser 200 to move between a load (Figure 21) and delivery position (Figure 20) corresponding to the positions of the actuation arm 112.

The actuation means 210 includes rack 220 and gear 221 assemblies. The rack 220 includes a button member 219 that is manually slidable within guide surfaces of housing 202 between an "armed" position shown in Figure 21 and a release position shown in Figure 20. When the button member 219 is slid to the armed position, the gear 221 rotates clockwise in the drawing and causes a piston member in the pressurization means 211 to compress fluid (e.g. air) within the pressure chamber 122 of the cartridge. Alternatively, the rack 220/button member 219 may be replaced with a circular gear (not shown) connected to and driven by a pivotal mouthpiece cover (not shown).

Clockwise rotation of the gear 221 in Figure 20 also causes gear 276 to rotate spring drive clutch 275. Spring drive clutch 275 engages one-way gear clutch 162 to drive the blades 160 within the medicament reservoir 111 in a predetermined powder loading direction and releases and does not drive the one-way gear clutch 162 when the gear 221 rotates counter-clockwise in Figure 20.

At generally the same time that the button member 219 is slid to the armed position, a linkage 225 causes the cammed fork member 204 to pivot about its pivot point on housing 202 against the bias of firing spring 226. The linkage 225 will cause the cammed fork member 204 to pivot until just after the fork member 204 engages cam surfaces 231 on latch member 230. The latch member 230 is pivotally mounted on the housing 202 for movement between a latched and release position. The latch member 230 includes a latching spring 235 for biasing the latch member 230 toward the latched position, and a release button 238 for manually pivoting the latch member 230 against the bias of the spring 235.

When the cammed fork member 204 engages cam surfaces 231, the latch member 230 pivots against the bias of spring 235 out of the path of the cammed fork member 204 until a trailing edge of the cammed fork member 204 clears the cam surfaces 231 then engages shoulder surfaces 239 of latch 230. This is the position of the cammed fork member 204 and the latch 230 shown in Figure 21.

The linkage 225 causes the actuation arm 112 to move from the delivery to the load position before the button 219 moves completely to the armed position. During a final portion of the movement of the button 219 to the armed position, the actuation arm 112 will be in the delivery position and the spring drive clutch 275 will cause the blades 160 to load the dosage chamber 131 with medicament.

After the dosage chamber 131 is loaded with a dosage of medicament and after the actuation arm 112/cammed fork member 204 is in the position shown in Figure 21, the dispenser is ready for actuation. To actuate the dispenser 200, a user manually presses on the button 238 which releases cammed fork member 204 which is under the bias of spring 226. The spring 226 moves the cammed fork member 204 from the load to the delivery position and consequently the actuation arm 112 from the load to the delivery position. It should be noted that the latch member 230 need not be a button member but may instead comprise a breath actuated means such as the breath actuated means shown in U.S. Patents 5,069,204; 4,664,107; and those mentioned in 4,664,107 including 3,187,748; 3,456,644; 3,645,645; 3,456,646; 3,565,070; 3,598,294; 3,814,297; 3,605,738; 3,732,864; 3,636,949; 3,789,843 and 3,187,748.

Referring now to Figure 21, the linkage 225 comprise a slider member at its distal end. The linkage 225 may be constructed to move the gear 221 and the button 219 back to the position shown in Figure 20 after the button 238 is pressed. Alternatively, the linkage 225 may be constructed to release after the button 238 is pressed. Instead, the user may manually move the button 219 from the position shown in Figure 21 to the position shown in Figure 20 after the cartridge 100 is fired.

## Claims

1. A dry powder medicament dispenser (10) comprising:
a housing (14) including a mouthpiece portion (15) adapted to be received in a user's mouth, said housing (14) having:
a medicament reservoir (11) adapted for storing dry powder medicament,
a medicament delivery passageway (16) opening through said mouthpiece portion (15),
a gas pressure chamber (22) including a pressure outlet passageway (23) generally adjacent said medicament delivery passageway (16),
means (24) for pressurizing gas within the gas pressure chamber (22) above ambient pressure,
a dosage member (3) comprising a sealing surface (33) and having a dosage chamber (32),
means mounting said dosage member (30) for movement between {1} a load position with the dosage chamber (32) opening into the medicament reservoir (11) and with the sealing surface of the dosage member (30) sealing the pressure outlet passageway to seal pressure within the gas pressure chamber (22), to {2} a delivery position with the dosage chamber (32) extending between the pressure outlet passageway (23) and the medicament delivery passageway (16).

2. A dispenser (10) according to claim 1 wherein progressively increasing portions of the pressure outlet passageway (23), the dosage chamber (32), and the medicament delivery passageway (16) are adapted to move into alignment during movement of the dosage member (30) from the load position to the delivery position to afford dispersion of the dry powder medicament.

3. A dispenser (10) according to claim 1 or 2 wherein said dispenser further includes means for transferring powder from the medicament reservoir (11) to said dosage chamber (32).

4. A dispenser according to claim 3 wherein said means for transferring powder comprises a flexible powder loading blade (36), and said dispenser (10) further includes means mounting said blade (36) for movement across said dosage chamber (32) such that the blade (36) progressively increasingly bends as it moves across the dosage chamber (32).

## Patentansprüche

1. Trockenpulvermedikamentenspender (10) mit:
einem Gehäuse (14), das einen zur Aufnahme in den Mund eines Benutzers geeigneten Mundstückabschnitt (15) aufweist, mit:
einem zur Aufbewahrung eines Trockenpulvermedikaments geeigneten Medikamentenreservoir (11),
einem Medikamentenabgabekanal (16), der durch den Mundstückabschnitt (15) führt,
einer Gasdruckkammer (22), die einen Druckauslaßkanal (23) aufweist, der allgemein an den Medikamentenabgabekanal (16) angrenzt,
einer Einrichtung (24), um Gas innerhalb der Gasdruckkammer (22) auf einen Druck oberhalb des Umgebungsdrucks zu setzen,
einem Dosierelement (30), das eine Dichtungsfläche (33) aufweist und eine Dosierkammer (32) hat,
einer Einrichtung zum Anordnen des Dosierelements (30) für eine Bewegung zwischen (I) einer Ladeposition, wobei die Dosierkammer (32) zum Medikamentenreservoir (11) hin offen ist und wobei die Dichtungsfläche des Dosierelements (30) den Druckauslaßkanal abdichtet, um den Druck innerhalb der Gasdruckkammer (22) abzudichten, und einer (II) Abgabeposition, wobei die Dosierkammer (32) sich zwischen dem Druckauslaßkanal (23) und dem Medikamentenabgabekanal (16) erstreckt.

2. Spender (10) nach Anspruch 1, wobei während der Bewegung des Dosierelements (30) von der Ladeposition in die Abgabeposition allmählich zunehmende Abschnitte des Druckauslaßkanals (23), der Dosierkammer (32) und des Medikamentenabgabekanals (16) ausgestaltet sind, um sich in eine Ausrichtung zu bewegen, um eine Verteilung des Trockenpulvermedikaments zu erlauben.

3. Spender (10) nach Anspruch 1 oder 2, wobei der Spender ferner eine Einrichtung zum Übertragen des Pulvers von dem Medikamentenreservoir (11) in die Dosierkammer (32) aufweist.

4. Spender nach Anspruch 3, wobei die Einrichtung zum Übertragen des Pulvers ein biegsames Pulverladeblatt (36) aufweist und der Spender (10) ferner eine Einrichtung aufweist, um das Blatt (36) für eine Bewegung über die Dosierkammer (32) anzubringen, so daß sich das Blatt (36) allmählich zunehmend biegt, wenn es sich über die Dosierkammer (32) bewegt.

## Revendications

1. Distributeur de médicament en poudre sèche (10) comprenant :
une enveloppe (14) comprenant une partie d'embout (15) adaptée pour être logée dans la bouche d'un utilisateur, ladite enveloppe (14) comportant :
un réservoir à médicament (11) adapté pour conserver un médicament sous forme de poudre sèche,
un passage de distribution de médicament (16) s'ouvrant dans ladite partie d'embout (15),
une chambre à pression de gaz (22) comprenant un passage de sortie à pression (23) généralement adjacent audit passage de distribution de médicament (16),
un moyen (24) pour pressuriser du gaz à l'intérieur de la chambre à pression de gaz (22) au-dessus de la pression ambiante,
un organe de dosage (30) comprenant une surface de scellage (33) et comportant une chambre de dosage (32),
un moyen de montage de l'organe de dosage (30) pour un déplacement entre (1) une position de chargement avec la chambre de dosage (32) s'ouvrant dans le réservoir à médicament (11) et avec la surface de scellage de l'organe de dosage (30) fermant le passage de sortie à pression pour obturer la pression à l'intérieur de la chambre à pression de gaz (22), et (2) une position de distribution avec la chambre de dosage (32) s'étendant entre le passage de sortie à pression (23) et le passage de distribution de médicament (16).

2. Distributeur (10) suivant la revendication 1, dans lequel des parties augmentant progressivement du passage de sortie à pression (23), de la chambre de dosage (32) et du passage de distribution de médicament (16) sont adaptées pour se déplacer en alignement au cours du déplacement de l'organe de dosage (30) de la position de chargement à la position de distribution pour offrir une dispersion du médicament en poudre sèche.

3. Distributeur (10) suivant l'une ou l'autre des revendications 1 et 2, dans lequel ledit distributeur comprend de plus un moyen pour transférer de la poudre du réservoir à médicament (11) à la chambre de dosage (32) précitée.

4. Distributeur suivant la revendication 3, dans lequel ledit moyen pour transférer de la poudre comprend une lamelle de chargement de poudre flexible (36) et ledit distributeur (10) comprend de plus un moyen de montage de ladite lamelle (36) pour un déplacement à travers ladite chambre de dosage (32) de telle sorte que la lamelle (36) se fléchisse progressivement de plus en plus lorsqu'elle se déplace à travers la chambre de dosage (32).
